Europäisches Patentamt
European Patent Office
Office européen des brevets

(11) Veröffentlichungsnummer: **0 303 296 A2**

# (12) EUROPÄISCHE PATENTANMELDUNG

(21) Anmeldenummer: **88113177.5**

(22) Anmeldetag: **12.08.88**

(51) Int. Cl.4: **A61K 31/765 , A61K 31/78**

Patentansprüche für folgende Vertragsstaaten: ES + GR.

(30) Priorität: **14.08.87 DE 3727082**

(43) Veröffentlichungstag der Anmeldung: **15.02.89 Patentblatt 89/07**

(84) Benannte Vertragsstaaten: **AT BE CH DE ES FR GB GR IT LI LU NL SE**

(71) Anmelder: **GÖDECKE AKTIENGESELLSCHAFT**
**Salzufer 16**
**D-1000 Berlin 10(DE)**

(72) Erfinder: **Osswald, Hartmut, Prof., Dr.**
**Kiefernweg 1**
**D-7808 Waldkirch 2(DE)**
Erfinder: **Betche, Hans-Jürgen, Dr.**
**Im Gottesacker 8**
**D-7801 Vörstetten(DE)**
Erfinder: **Hartenstein, Johannes, Dr.**
**Fohrenbühl 23**
**D-7801 Stegen-Wittental(DE)**
Erfinder: **Stoll, Gerhard, Dr.**
**Danzigerstrasse 69**
**D-4052 Korschenbroich 1(DE)**
Erfinder: **Weinheimer, Günter, Dr.**
**Sachsenstrasse 4**
**D-7809 Denzlingen(DE)**

## (54) Pharmazeutische Zubereitungen zur Behandlung der Urolithiasis.

(57) Die Erfindung betrifft parenterale pharmazeutische Zubereitungen, welche wasserlösliche oder kolloidal wäßrig lösliche polymere oder copolymere Alkancarbonsäuren mit einem Molgewicht von>5.000 enthalten, die nach dem Schema

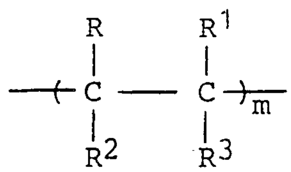

(I),

hergestellt worden sind. Die neuartigen pharmazeutischen Zubereitungen eignen sich zur Bekämpfung der Urolithiasis.

EP 0 303 296 A2

## Pharmazeutische Zubereitungen zur Behandlung der Urolithiasis

Gegenstand der Erfindung sind parenterale pharmazeutische Zubereitungen, die mindestens eine wasserlösliche oder kolloidal wäßrig lösliche polymere Alkancarbonsäure der allgemeinen Formel I enthalten,

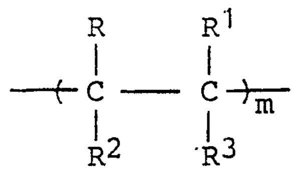 (I),

in welcher die Reste R, $R^1$, $R^2$, und $R^3$, die gleich oder verschieden sein können, ein Wasserstoff- oder Halogenatom, eine C1-C4 Alkyl- oder C2-C4 Alkenylgruppe, einen Nitrilrest, eine C1-C5 Alkoxycarbonyl-, Acetoxy- oder Aminocarbonylgruppe, eine gegebenenfalls durch 1-2 Halogenatome oder Hydroxylgruppen substituierte Phenyl- oder Styrylgruppe, eine Carboxymethyl-, Carboxyethyl- oder Carboxy-C2-C4-alkenylgruppe oder einen Cycloalkylrest mit 3 bis 8 Kohlenstoffatomen, wobei zwei der Reste R, $R^1$, $R^2$ und $R^3$ gemeinsam auch eine Anhydridgruppe bilden können und wobei m eine ganze Zahl zwischen 100 und 200.000 bedeutet, mit der zusätzlichen Maßgabe, daß die polymere Alkancarbonsäure als Reste R, $R^1$, $R^2$, oder $R^3$ eine für die Wasserlöslichkeit, bzw. die kolloidal wäßrige Löslichkeit ausreichende Anzahl Carboxylgruppen oder deren Salze enthält.

Als Halogenatome kommen Fluor, Chlor und Brom, bevorzugt Chlor und Brom in Frage.

Das mittlere Molekulargewicht der Verbindungen I liegt im Falle von Homopolymeren bei 10.000 bis 3.000.000, vorzugsweise bei 30.000 bis 500.000, im Falle von Copolymeren kann jedoch bereits bei einem Molekulargewicht von 5.000 eine brauchbare Wirksamkeit vorliegen.

Die Verbindungen I können aus gleichartigen Monomeren der allgemeinen Formel II

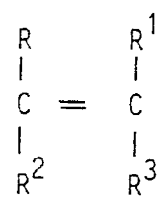 (II),

in welcher die Reste R, $R^1$, $R^2$, und $R^3$ die oben genannte Bedeutung haben, in Form von Homopolymeren, oder aus verschiedenartigen Monomeren als Copolymere zusammengesetzt sein. Die Verbindungen I sind bekannt und werden nach bekannten Polymerisationsverfahren hergestellt. Die Carboxylgruppen (-COOH) können auch durch Hydrolyse funktioneller Gruppen nach der Polymerisation freigesetzt werden. Funktionelle Gruppen sind beispielsweise Nitril-, Amid- oder Estergruppen.

Copolymere Alkancarbonsäuren können auch Anteile aus Monomeren enthalten, die keine Carboxylgruppe besitzen. Der Anteil des keine Carboxylgruppe aufweisenden Monomeren am Copolymeren I richtet sich ebenfalls nach der erforderlichen Wasserlöslichkeit der erfindungsgemäßen Polyalkancarbonsäuren oder ihrer Salze und liegt vorzugsweise bei 20-60 Mol%. Enthalten alle Komponenten Carboxylgruppen, ist der Anteil je nach der erzielbaren Löslichkeit frei wählbar. Im Falle gleichartiger Monomeren (Homopolymeren) stellt aber jeweils mindestens einer der Reste R, $R^1$, $R^2$ oder $R^3$ eine Carboxylgruppe dar.

Die Häufigkeit von Nierensteinerkrankungen variiert stark in den verschiedenen Teilen der Welt, erreicht aber in einigen Ländern einen ebenso hohen Häufigkeitsgrad wie Diabetes mellitus. Aufgrund ernährungsbedingter Faktoren ist die Zahl der Erkrankungen steigend. Die chemische Analyse der Nierensteine zeigt, daß ungefähr 70% dieser Steine aus Calciumoxalat bestehen.

Die gegenwärtigen Methoden zur Beseitigung von Nierensteinen liegen in der operativen Entfernung, der Zertrümmerung durch extrakorporale Stoßwellenlithotripsie und ihrer Auflösung durch langwierige Spülungen mit chelatisierenden Agentien. Da alle diese Behandlungsmethoden mit erheblichen Nebenwirkungen verbunden sind, und da die Nierensteinkrankheit mit einer Rezidivrate von 50-60% einhergeht,

besteht ein dringender Bedarf nach einer medikamentösen Pro- bzw. Metaphylaxe. Eine erfolgreiche medikamentöse Therapie führt außerdem zu einer deutlichen Senkung der Behandlungskosten (J. Urol. 134, 6-10, 1985). Die derzeitige medikamentöse Therapie der Nierensteinkrankheit strebt eine Verminderung der Konzentration der Soluta im Urin an, die zur Übersättigung des Urins wesentlich beitragen und damit Risikofaktoren der Steinbildung sind.

Die praktizierten Methoden zur medikamentösen Prophylaxe von Nierensteinen reichen von Trinkkuren bis zum Einsatz von systemisch wirksamen Arzneimitteln. Die Ergebnisse sind jedoch nicht befriedigend. Bei der Nierensteinprophylaxe durch die sogenannte Alkali-Therapie, überwiegend durchgeführt mit Alkalicitraten, führt die Alkalisierung des Harns zu einem erheblichen Risiko der Phosphatsteinbildung. Außerdem stellt die hohe Dosis von täglich 9-12 g Citrat eine hohe Belastung für den Patienten dar, woraus folgt, daß die Patienten diese prophylaktische Therapie kaum länger als 6-8 Wochen durchhalten.

Die Behandlung der Urolithiasis mit Thiazid-Diuretika kann zu unerwünschten Nebenwirkungen wie Hypokaliämie, Glykosurie und Hyperurikämie führen. Als weiteres therapeutisches Prinzip zur Prophylaxe der Urolithiasis wurde auch vorgeschlagen, daß zur Bindung und Verminderung von resorbierbarem Calcium im Darm chelatbildende Polyelektrolyte wie Natriumcellulosephosphat (New England J. Med. 290, 175, 1974), Polystyrolsulfonsäuren (EP-A-0 068 453), polymere Zuckersäuren (DE-A- 33 32 301), Kationenaustauscher (GB-A-1 498 101) und auch Polyacrylate (US-A-4 143 130) oral verabreicht werden. Dabei reduzieren diese Stoffe im Darm, ohne selbst resorbiert zu werden, die Calciumaufnahme aus der Nahrung durch die Darmschleimhaut.

Diese Methoden können aber zu einem nicht unerheblichen Eingriff in den Calciumhaushalt des Körpers mit der Gefahr einer unzureichenden Mineralisation infolge Calciumentzugs führen. Diese Therapie der Verminderung der intestinalen Calciumresorption ist nur sinnvoll bei der idiopathischen Hypercalciurie und wirkt dabei nur bei einem sehr kleinen Prozentsatz der Nierensteinkranken. Entsprechend beobachtet man in einem Modellversuch bei Ratten, die einer lithogenen, zu einer überschüssigen Oxalatbildung führenden Diät ausgesetzt wurden, nach oraler Applikation von Polyacrylaten keinerlei Effekt auf die Bildung von Calciumoxalat-Nierensteinen.

Überraschenderweise wurde nun gefunden, daß Polyalkancarbonsäuren der Formel I mit einem Molekulargewicht von 5.000 und höher bei parenteraler Gabe die Bildung von Calciumoxalatnierensteinen in vivo hemmen. Unterhalb von einem Molekulargewicht von 10.000 ist die Wirksamkeit auf Einzelfälle von Copolymerisaten beschränkt.

Es ist zwar schon in der Literatur (Austr. J. Chem. 21, 1067, 1968) berichtet worden, daß anionische Polyelektrolyte in vitro die Kristallisation von Calciumoxalat inhibieren. Andererseits ergaben diese Untersuchungen aber auch, daß die Wirksamkeit der synthetischen Polyelektrolyte mit abnehmendem Molekulargewicht zunimmt. Die wirksamsten Verbindungen besaßen dabei ein Molekulargewicht von 2-3000. Vergleichsuntersuchungen über den Einfluß von Polyalkancarbonsäuren mit unterschiedlichen Molekulargewichten auf die Kristallisation von Calciumoxalat in einer kontinuierlich betriebenen Kristallisationskammer ergaben keine wesentlichen Unterschiede bezüglich Kristallisation, Wachstumsrate und Kristallaggregation. Im Invivo-Modell mit Polyacrylsäuren (Homopolymerisat) zeigten diese niedermolekularen Polyalkancarbonsäuren (MG<10.000) keine Inhibierung der Nierensteinbildung. Der Befund, daß Polyalkancarbonsäuren mit hohem Molekulargewicht in vivo die Nierensteinbildung inhibieren, muß daher als unerwartet angesehen werden.

Die erfindungsgemäßen polymeren Alkancarbonsäuren besitzen ein mittleres Molekulargewicht im Bereich von 10.000 bis 3.000.000 (Homopolymerisate) sowie 5.000 bis 5.000.000 (Copolymerisate), wobei der bevorzugte Bereich im Falle der Polyacrylate zwischen 30.000 und 500.000 liegt. Bevorzugte Verbindungen sind Polyacrylsäuren und Polymethacrylsäuren sowie Copolymere aus Maleinsäure mit Ethylen oder Methylvinylether sowie Copolymere aus Acrylsäure mit Methacrylsäure oder Maleinsäure.

Die wasserlöslichen polymeren Alkancarbonsäuren bzw. deren Salze, die in dieser Erfindung Anwendung finden, sind meist handelsübliche Verbindungen, die nach literaturbekannten Verfahren (Houben Weyl, Methoden der organischen Chemie, 4. Auflage, Band 14/1, S. 1010 ff, US-A-2 744 098, US-A-4 143 130) hergestellt werden können. Sie werden in bekannter Weise z.B. durch Bulk-, Suspensions-, Lösungs-oder Emulsionspolymerisation hergestellt oder durch Hydrolyse bzw. Teilhydrolyse von polymeren Alkancarbonsäurederivaten erhalten. Je nach Polymerisationsmethode können auch unterschiedliche, nichttoxische und inerte Endgruppen oder bei ggf. erfolgten Hydrolysen nicht hydrolysierte Carbonsäurederivate im erfindungsgemäßen Produkt enthalten sein. Die Tabelle IV enthält eine Aufstellung von Komponenten (II) der polymeren Alkancarbonsäuren I.

Die erfindungsgemäß beanspruchten polymeren Alkancarbonsäuren finden bevorzugt Anwendung in Form ihrer Salze, wobei darauf geachtet werden muß, daß die wäßrige oder kolloidal wäßrige Lösung dieser Salze einen für parenterale Applikation geeigneten pH-Wert besitzt. Typische nichttoxische und

pharmazeutisch annehmbare Salze, die in dieser Erfindung Anwendung finden, sind Alkalisalze sowie Ammonium- und Aminsalze. Die pro 24 Stunden bioverfügbaren Dosen der erfindungsgemäßen Verbindungen sollten zwischen 5 - 100 mg liegen. Dementsprechend sollten die Lösungen mindestens eine Tagesdosis der Verbindungen I pro 100 ml enthalten. Die Löslichkeit der pharmazeutischen Zubereitungen im Falle von Depot- oder transdermalen Formen muß natürlich ebenfalls so eingestellt werden, daß eine Tagesdosis von mindestens 5 mg freigesetzt wird. Entsprechend der galenischen Zubereitung müssen die Dosen täglich oder in längeren Abständen, wie sie für protrahierte Wirkungen erfahrungsgemäß üblich sind, angewendet werden. Zum Erreichen der protrahierten Wirkung können auch subkutane Depotformen oder transdermale Systeme verwendet werden.

Die folgenden Vergleichsversuche zeigen die Wirksamkeit der erfindungsgemäßen Zubereitungen.

## Herstellung der Testzubereitungen

Die Herstellung der Zubereitungen erfolgt dadurch, daß man die polymeren Aklancarbonsäuren der allgemeinen Form zunächst in eine 10-60 Gew.%ige wäßrige Lösung überführt.

Die meisten Alkancarbonsäuren werden jedoch bereits in Form von konzentrierten wässrigen Lösungen im Handel angeboten, so daß man von diesen Lösungen ausgehen kann.

Man stellt dann eine applizierbare Lösung von 1-3 Gew.%, bevorzugt 0,8-1,8 Gew.% her, die nach Sterilfiltration unmittelbar injiziert werden kann.

Im Tierversuch werden 0,2-0,3 ml, maximal 1 ml pro Ratte verwendet.

## Beispiel

Eine 40 Gew.%ige wäßrige Lösung einer Polyacrylsäure mit einem Molekulargewicht von 70.000 bis 80.000 wurde bis zu einer Konzentration von 1 Gew.% verdünnt. Der pH-Wert wurde anschließend auf 5,0 eingestellt.

Zur Applikation von 10 mg/kg der Wertesubstanz waren somit 1 ml/kg erforderlich.

## VERGLEICHSVERSUCHE

I. Im ersten Schritt einer Untersuchung zur Hemmung von Calciumoxalatkristallbildung wurde eine Kristallisationskammer verwendet, die die Kristallisation von übersättigtem Calciumoxalat in synthetischem Urin analysiert. Die Kristallisationskammer hat ein Volumen von 70 ml und enthält folgende Ionen (Konzentration in mmol/l): Calcium (6,0), Magnesium (3,0), Natrium (185), Kalium (81), Ammoniumion (43,4), Chlorid (252), primäres Phosphat (22), sekundäres Phosphat (3.0), Sulfat (20), Citrat (1,99). Die Lösung hat bei 20 °C ein pH von 5,8. Bei einem Zu- bzw. Abfluß von 10 ml/min beträgt die mittlere Verweildauer der Lösung = 7 min. Nach 90 min Equilibrierungszeit unter 37 °C werden mit einem Coulter-Counter Typ TA 11 2 ml der Lösung abgesaugt und in den Kanälen 1-16 so analysiert, daß man die Partikelgröße von gebildeten Kristallen ablesen kann. Aus der Verteilung der Partikelgröße und der mittleren Verweildauer der Lösung in der Kristallisationskammer lassen sich berechnen die Wachstumsrate der Kristalle in µm/min, die Nukleationsrate, ausgedrückt in Anzahl (n) von Mikrokristallen pro ml Lösung und Minute sowie durch graphische Extrapolation die Zahl der Partikel mit entsprechendem Durchmesser über den Bereich einer Kristallgröße von 0-50 µm Durchmesser. Die Theorie der Kristallbildung, der Berechnungen von Nukleationsrate, Wachstumsrate und Kristallkonzentration in der Kristallisationskammerflüssigkeit ist von Roberton et al. und Mitarbeitern 1983 ausführlich dargestellt worden.

II. Zur Prüfung der Wirksamkeit einer Substanz auf Calciumoxalatkristallbildung in der Niere wird ein Versuchsmodell an Ratten eingesetzt. Dabei erhalten die Tiere eine Vitamin $B_6$-arme Diät (Althromin C 1023) für drei Tage sowie ein Trinkwasser, dem 1 g Äthylenglykol pro 100 ml Leitungswasser zugesetzt werden. Nach 3-7 Tagen entwickeln die Tiere eine disseminierte reine Calciumoxalatkristallbesiedlung des Nierenparenchyms. Die Abwesenheit einer Phosphatkontamination dieser Kristalle nach der vorgegebenen Diät wurde früher untersucht und mitgeteilt (Ernst, Osswald, 1986).

Zur Inspektion der disseminierten Kristallabsiedlung in der Niere werden die Ratten nach 7 Tagen getötet,

die Nieren werden entnommen durch Schnittführung an der größen Circumferenz halbiert und unter einem Stereomikroskop mit 50 - 150-facher Vergrößerung semiquantitativ befundet.
Die Substanzen, die eine Verhinderung der Kristallbildung bewirken sollen (Prophylaxe), werden 2 Tage vor Beginn der oben beschriebenen Diät den Tieren verabreicht, entweder durch subkutane Injektion oder über eine subkutan implantierte Alzetpumpe.

TABELLE I:

| Beispiele für die auf Inhibierung von Calciumoxalat-Nierensteinen geprüften Alkanpolycarbonsäuren | | |
|---|---|---|
| Beispiele | Zusammensetzung | MG |
| A | PAS | 1.040 |
| B | PAS | 2.000 |
| C | PAS | 2.000 - 5.000 |
| D | PAS | 2.400 |
| E | PAS | 3.500 |
| F | PAS | 4.000 |
| G | PAS | 5.000 |
| H | PAS | 8.000 |
| I | PAS | 17.000 |
| J | PAS | 35.000 - 40.000 |
| K | PAS | 38.000 |
| L | PAS | 60.000 |
| M | PAS | 70.000 |
| N | PAS | 70.000 - 80.000 |
| O | PAS | 90.000 |
| P | PAS | 150.000 - 200.000 |
| Q | PAS | 180.000 |
| R | PAS | 250.000 |
| S | PAS | 300.000 - 400.000 |
| T | PAS | 400.000 - 500.000 |
| U | PMAS | 8.000 |
| V | PMAS | 10.000 |
| W | PMAS | 80.000 |
| X | AS:MS-Co (4:1) | 6.000 |
| Y | AS:MAS-Co (1:1) | 70.000 |
| Z | MAS:MME-Co (2:1) | 50.000 - 1.000.000 |
| AA | E:MS-Co (1:1) | 13.000 |
| AB | E:MS-Co (1:1) | 40.000 |
| AC | E:MS-Co (1:1) | 160.000 |
| AD | MVE:MS-Co (1:1) | 150.000 |
| AE | MVE:MS-Co (1:1) | 450.000 |
| AF | MVE:MS-Co (1:1) | 1.200.000 |
| AG | MVE:MS-Co (1:1) | 3.000.000 |
| PAS Polyacrylsäure MS Maleinsäure<br>PMAS Polymethacrylsäure E Ethylen<br>AS Acrylsäure MVE Methylvinylether<br>MAS Methacrylsäure MG mittleres Molekulargewicht<br>Co Copolymer MME Methacrylsäuremethylester | | |

TABELLE II:

| Inhibierung der Calciumoxalatsteinbildung in der Kristallisationskammer. Die Anzahl der Experimente beträgt - sofern nicht gesondert vermerkt - n = 2 | | | |
|---|---|---|---|
| Beispiele | Molekulargewicht | Konzentration mol/l | Wachstumsrate G, µm/min |
| Kontrolle | - | | 0,388 |
| Na-Citrat | 192 | $1 \times 10^{-3}$ | 0,376 |
| | | $5 \times 10^{-3}$ | 0,310 |
| B | 2000 | $2 \times 10^{-7}$ | 0,220 |
| C | 2-5000 | $2.6 \times 10^{-5}$ | 0,154 |
| F | 4000 | $10^{-6}$ | 0,149 |
| G | 5000 | $10^{-6}$ | 0,173 |
| H | 8000 | $10^{-6}$ | 0,131 |
| I | 17000 | $10^{-6}$ | 0,147 |
| J | 35-40000 | $10^{-6}$ | 0,114 |
| K | 38000 | $10^{-6}$ | 0,119 |
| L | 60000 | $10^{-6}$ | 0,138 |
| N | 70-80000 | $1 \times 10^{-8}$ | 0,150 |
| O | 90000 | $10^{-6}$ | 0,167 |
| S | 300-400000 | $10^{-6}$ | 0,172 |
| AA | 13000 | $10^{-6}$ | 0,148 |
| AB | 40000 | $10^{-5}$ | 0,138 |
| AC | 160000 | $10^{-6}$ | 0,138 |
| AE | 450000 | $10^{-6}$ | 0,113 |
| AF | 1200000 | $10^{-6}$ | 0,134 |
| AG | 300000 | $10^{-6}$ | 0,070 |

TABELLE III:

| Inhibierung der Calciumoxalatsteinbildung in dem in vivo Modell. Jede Dosis wurde an 6 Tieren getestet. | | | |
|---|---|---|---|
| Bewertung der prophylaktischen Wirksamkeit: + = 20-40 % Hemmung<br>+ + = 50-70 % Hemmung<br>+ + + = 90-100 % Hemmung<br>- = ohne Wirkung | | | |
| Beispiele | Molekulargewicht | Dosis mg/kg | Bewertung |
| B | 2.000 | 30 | - |
| C | 2.000-5.000 | 30 | - |
| G | 5.000 | 30 | - |
| H | 8.000 | 30 | - |
| I | 17.000 | 10 | + |
| | | 30 | + + |
| J | 35.000-40.000 | 30 | + + + |
| K | 38.000 | 10 | + + |
| | | 30 | + + + |
| L | 60.000 | 10 | + + + |
| | | 30 | + + + |
| N | 70.000-80.000 | 12 | + + + |
| O | 90.000 | 7,5 | + + |
| | | 25 | + + |
| S | 300.000-400.000 | 3 | + + |
| | | 10 | + + + |
| AA | 13.000 | 30 | + + + |
| AB | 40.000 | 30 | + + + |
| AC | 160.000 | 30 | + + + |
| AE | 450.000 | 30 | + |
| AF | 1.200.000 | 30 | + + |
| AG | 3.000.000 | 30 | + + + |

TABELLE IV

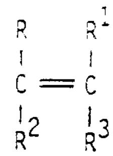

| | R | $R^1$ | $R^2$ | $R^3$ |
|---|---|---|---|---|
| Acrylsäure | H | COOH | H | H |
| Methacrylsäure | H | COOH | H | $CH_3$ |
| Ethacrylsäure | H | COOH | H | $C_2H_5$ |
| α-Chloracrylsäure | H | COOH | H | Cl |
| α-Cyanoacrylsäure | H | COOH | H | CN |
| Crotonsäure | H | COOH | $CH_3$ | H |
| Hydrosorbinsäure (trans-2-Hexensäure) | H | COOH | n-$C_3H_7$ | H |
| Sorbinsäure | H | COOH | HC=CH-$CH_3$ | H |
| α-Chlorsorbinsäure | H | COOH | HC=CH-$CH_3$ | Cl |
| Zimtsäure | H | COOH | $C_6H_5$ | H |
| β-Styrylacrylsäure (1-Carboxy-4-phenyl-butadien-1,3) | H | COOH | HC=CH-$C_6H_5$ | H |
| Muconsäure | H | COOH | CH=CH-COOH | H |
| Hydromuconsäure (trans-2-Hexendisäure) | H | COOH | $CH_2$-$CH_2$-COOH | H |
| Itaconsäure | H | COOH | H | $CH_2$-COOH |
| Citraconsäure | COOH | COOH | H | $CH_3$ |
| Mesaconsäure | H | COOH | COOH | $CH_3$ |
| Trans-Glutaconsäure | H | COOH | $CH_2COOH$ | H |
| Cis-Aconitsäure | COOH | COOH | $CH_2COOH$ | H |
| Trans-Aconitsäure | $CH_2COOH$ | COOH | COOH | H |
| α-Phenylacrylsäure | H | COOH | H | $C_6H_5$ |
| α-Butylcrotonsäure | H | COOH | H | n-$C_4H_9$ |
| Seneciosäure | $CH_3$ | COOH | $CH_3$ | H |
| Angelicasäure | $CH_3$ | COOH | H | $CH_3$ |
| Tiglinsäure | H | COOH | $CH_3$ | $CH_3$ |
| m-Chlor-Zimtsäure | H | COOH | m-CI-$C_6H_4$ | H |
| p-Chlor-Zimtsäure | H | COOH | p-CI-$C_6H_4$ | H |
| Umbellsäure | H | COOH | 2,4-$(OH)_2$-$C_6H_3$ | H |
| Maleinsäure | COOH | COOH | H | H |
| Fumarsäure | H | COOH | COOH | H |
| Tricarboxyethylen | COOH | COOH | COOH | H |
| Tetracarboxyethylen | COOH | COOH | COOH | COOH |
| Ethylen | H | H | H | H |
| Methylvinylether | H | $OCH_3$ | H | H |
| Vinylacetat | H | $OCOCH_3$ | H | H |
| Styrol | H | $C_6H_5$ | H | H |

**Ansprüche**

1. Parenterale pharmazeutische Zubereitung enthaltend mindestens eine wasserlösliche oder kolloidal wäßrig lösliche polymere Alkancarbonsäure der allgemeinen Formel I

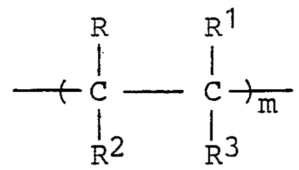
(I),

in welcher die Reste R, $R^1$, $R^2$, und $R^3$, die gleich oder verschieden sein können, ein Wasserstoff- oder Halogenatom, eine C1-C4 Alkyl- oder C2-C4 Alkenylgruppe, einen Nitrilrest, eine C1-C5 Alkoxycarbonyl-, Acetoxy- oder Aminocarbonylgruppe, eine gegebenenfalls durch 1-2 Halogenatome oder Hydroxylgruppen substituierte Phenyl- oder Styrylgruppe, eine Carboxymethyl-, Carboxyethyl- oder Carboxy-C2-C4-alkenylgruppe oder einen Cycloalkylrest mit 3 bis 8 Kohlenstoffatomen, wobei zwei der Reste R, $R^1$, $R^2$ und $R^3$ gemeinsam auch eine Anhydridgruppe bilden können und wobei m eine ganze Zahl zwischen 100 und 200.000 bedeutet, mit der zusätzlichen Maßgabe daß die polymere Alkancarbonsäure als Reste R, $R^1$, $R^2$, oder $R^3$ eine für die Wasserlöslichkeit, bzw. die kolloidal wäßrige Löslichkeit ausreichende Anzahl Carboxylgruppen oder deren Salze enthält.

2. Pharmazeutische Zubereitung nach Anspruch 1, dadurch gekennzeichnet, daß die polymere Alkancarbonsäure ein Homopolymerisat ist.

3. Pharmazeutische Zubereitung nach Anspruch 1, dadurch gekennzeichnet, daß die polymere Alkancarbonsäure ein Copolymerisat ist.

4. Pharmazeutische Zubereitung nach Anspruch 1 bis 3, dadurch gekennzeichnet, daß die polymere Carbonsäure im Falle von Homopolymerisaten ein mittleres Molekulargewicht von 10.000 bis 3.000.000 und im Falle von Copolymerisaten ein mittleres Molekulargewicht von 5.000 bis 3.000.000 aufweist.

5. Pharmazeutische Zubereitung nach Anspruch 1 bis 4, dadurch gekennzeichnet, daß die polymere Alkancarbonsäure als Carboxylgruppen tragende monomere Komponenten Maleinsäure, Methacrylsäure oder Acrylsäure und als monomere Komponenten ohne freie Carboxylgruppen Ethylen, Methylvinylether oder Methacrylsäuremethylester enthält.

6. Pharmazeutische Zubereitung nach Anspruch 1 bis 5, dadurch gekennzeichnet, daß der Anteil der nicht Carboxylgruppen tragenden Komponenten in einer copolymeren Alkancarbonsäure 20 - 60 Mol % beträgt.

7. Verwendung einer wasserlöslichen oder kolloidal wäßrig löslichen polymeren Alkancarbonsäure gemäß Anspruch 1 bis 6 zur Herstellung von parenteralen pharmazeutischen Zubereitungen für die Bekämpfung der Urolithiasis.

Patentansprüche für die folgenden Vertragsstaaten: ES, GR

1.) Verwendung einer wasserlöslichen oder kolloidal wäßrig löslichen polymeren Alkancarbonsäure der allgemeinen Formel I

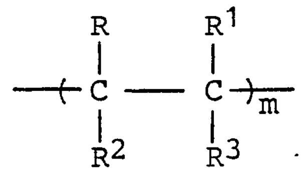
(I),

in welcher die Reste R, $R^1$, $R^2$, und $R^3$, die gleich oder verschieden sein können, ein Wasserstoff- oder Halogenatom, eine C1-C4 Alkyl- oder C2-C4 Alkenylgruppe, einen Nitrilrest, eine C1-C5 Alkoxycarbonyl-, Acetoxy- oder Aminocarbonylgruppe, eine gegebenenfalls durch 1-2 Halogenatome oder Hydroxylgruppen substituierte Phenyl- oder Styrylgruppe, eine Carboxymethyl-, Carboxyethyl- oder Carboxy-C2-C4-alkenylgruppe oder einen Cycloalkylrest mit 3 bis 8 Kohlenstoffatomen, wobei zwei der Reste R, $R^1$, $R^2$ und $R^3$ gemeinsam auch eine Anhydridgruppe bilden können und wobei m eine ganze Zahl zwischen 100 und 200.000 bedeutet, mit der zusätzlichen Maßgabe daß die polymere Alkancarbonsäure als Reste R, $R^1$, $R^2$, oder $R^3$ eine für die Wasserlöslichkeit, bzw. die kolloidal wäßrige Löslichkeit ausreichende Anzahl Carboxylgruppen oder deren Salze enthält, zur Herstellung einer parenteralen pharmazeutischen Zubereitung für die Bekämpfung der Urolithiasis.

2.) Verwendung nach Anspruch 1, dadurch gekennzeichnet, daß die polymere Alkancarbonsäure ein Homopolymerisat ist.

3.) Verwendung nach Anspruch 1, dadurch gekennzeichnet, daß die polymere Alkancarbonsäure ein Copolymerisat ist.

4.) Verwendung nach Anspruch 1 bis 3, dadurch gekennzeichnet, daß die polymere Carbonsäure im Falle von Homopolymerisaten ein mittleres Molekulargewicht von 10.000 bis 3.000.000 und im Falle von Copolymerisaten ein mittleres Molekulargewicht von 5.000 bis 3.000.000 aufweist.

5.) Verwendung nach Anspruch 1 bis 4, dadurch gekennzeichnet, daß die polymere Alkancarbonsäure als Carboxylgruppen tragende monomere Komponenten Maleinsäure, Methacrylsäure oder Acrylsäure und als monomere Komponenten ohne freie Carboxylgruppen Ethylen, Methylvinylether oder Methacrylsäuremethylester enthält.

6.) Verwendung nach Anspruch 1 bis 5, dadurch gekennzeichnet, daß der Anteil der nicht Carboxylgruppen tragenden Komponenten in einer copolymeren Alkancarbonsäure 20 - 60 Mol % beträgt.

7.) Herstellung einer pharmazeutischen Zubereitung enthaltend mindestens eine wasserlösliche oder kolloidal wäßrig lösliche polymere Alkancarbonsäure nach Anspruch 1 - 6, dadurch gekennzeichnet, daß man die Alkancarbonsäure in an sich bekannter Weise in eine 1-3 Gew.% enthaltende Lösung überführt und diese steril-filtriert.